(19) Europäisches Patentamt
European Patent Office
Office européen des brevets

(11) **EP 3 456 260 A1**

(12) **EUROPEAN PATENT APPLICATION**

(43) Date of publication:
**20.03.2019 Bulletin 2019/12**

(51) Int Cl.:
***A61B 5/085*** (2006.01)   ***A61B 5/091*** (2006.01)
***A61M 16/00*** (2006.01)

(21) Application number: **17191816.2**

(22) Date of filing: **19.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventor: **TE VELDE, Mart Kornelis-Jan**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **ESTIMATING A VOLUME OF LIQUID IN THE LUNGS OF A SUBJECT**

(57)    There is provided an apparatus (100) for estimating a volume of liquid in the lungs of a subject. The apparatus (100) comprises a control unit (110) configured to acquire a measurement indicative of one or both of a volume of gas in the lungs of the subject and a change in a volume of gas in the lungs of the subject. The control unit (110) is also configured to estimate the volume of liquid in the lungs of the subject from the acquired measurement.

<u>100</u>

Figure 1

EP 3 456 260 A1

**Description**

TECHNICAL FIELD OF THE INVENTION

The invention relates to the field of healthcare and, in particular, to estimating a volume of liquid in the lungs of a subject.

BACKGROUND TO THE INVENTION

Chronic heart failure (CHF) is a condition in which the heart is weakened and unable to circulate blood to meet the body's needs. The subsequent build-up of fluids in the legs, kidneys, and lungs characterises the condition as congestive. The weakening may be associated with either the left or right side or both sides of the heart, with different causes and treatments associated with each type. In most cases, it is the left side of the heart which fails, which means that the heart is unable to efficiently pump blood to the systemic circulation. As a result, blood starts to back up, which increases the pressure in the blood vessel and forces liquid from the blood vessels into body tissues. The ensuing fluid congestion of the lungs (namely, pulmonary congestion) results in changes in respiration, including alterations in rate and/or pattern, as well as changes in the volume of liquid in the lungs, since the extra liquid in the lungs makes it more difficult for the airways to expand as a person inhales. Thus, the change in the volume of liquid in the lungs provides a useful basis for assessing CHF status.

There are existing devices in the technical field for measuring or estimating a volume of liquid in the lungs of a subject for the purpose of monitoring CHF at home, which can be used by the subject themselves or medical personnel. These devices typically rely on techniques such as radar and bioelectrical impedance, or the use of a spirometer. For example, in some existing devices, a radar transceiver may be provided for transmitting radiofrequency energy at a targeted portion of the lungs so as to measure the dynamic motion of the lungs and subsequently determine a change in the volume of liquid in the lungs. In another example, bioelectrical impedance spectroscopy sensors maybe provided in order to measure fluid distribution in a targeted portion of the lungs. However, these devices are not widely used by CHF patients due to the low accuracy of the measurements. The devices also require a vest to be placed on the subject and user intervention is needed to place the vest on the subject for measurements to be taken, which can be obtrusive.

There is thus a need for an improved method and apparatus for accurately measuring or estimating a volume of liquid in the lungs of a subject.

SUMMARY OF THE INVENTION

As noted above, a limitation with existing approaches is a low level of accuracy of measurements of the volume of liquid in the lungs. It would thus be valuable to have an improved method and apparatus for estimating a volume of liquid in the lungs of a subject, which overcomes the existing problems.

Therefore, according to a first aspect, there is provided an apparatus for estimating a volume of liquid in the lungs of a subject. The apparatus comprises a control unit configured to acquire a measurement indicative of one or both of a volume of gas in the lungs of the subject and a change in a volume of gas in the lungs of the subject. The control unit is also configured to estimate the volume of liquid in the lungs of the subject from the acquired measurement.

In some embodiments, the volume of liquid in the lungs of the subject may be estimated from the acquired measurement by comparing the acquired measurement to one or more reference values.

In some embodiments, the volume of liquid in the lungs of the subject may be estimated to increase when the change in volume of gas in the lungs of the subject indicated by the acquired measurement is a decrease in the volume of gas in the lungs of the subject and the volume of liquid in the lungs of the subject maybe estimated to decrease when the change in volume of gas in the lungs of the subject indicated by the acquired measurement is an increase in the volume of gas in the lungs of the subject.

In some embodiments, the control unit may be configured to acquire the measurement from a gas flow sensor.

In some embodiments, the control unit may be configured to acquire the measurement by acquiring, from a pressure sensor, a measurement of pressure in the lungs of the subject in response to a delivery of a volume of gas to the lungs of the subject. In these embodiments, the measurement of the pressure may be indicative of one or both of the volume of gas in the lungs of the subject and a change in a volume of gas in the lungs of the subject.

In some embodiments, the measurement of pressure in the lungs of the subject may comprise a measurement of a rate at which a threshold pressure is detected in the lungs of the subject in response to delivery of the volume of gas to the lungs of the subject. In these embodiments, the measurement of the rate at which the threshold pressure is detected may be indicative of one or both of the volume of gas in the lungs of the subject and a change in a volume of gas in the lungs of the subject. In some embodiments, an increase in the rate at which the threshold pressure is detected may be indicative of a decrease in the volume of gas in the lungs of the subject and a decrease in the rate at which the threshold pressure is detected maybe indicative of an increase in the volume of gas in the lungs of the subject.

In some embodiments, the control unit may be configured to acquire the measurement by acquiring, from an acoustic sensor, an acoustic signal detected in response to delivery of the acoustic signal to the lungs of the subject, wherein the detected acoustic signal represents an acoustic response of the lungs. In some embodiments, the acoustic response may comprise one or more acoustic resonance frequencies indicative of one or both of the volume of gas in the lungs of the subject and a change in a volume of gas in the lungs of the subject. In some embodiments, an increase in the acoustic resonance frequency may be indicative of a decrease in the volume of gas in the lungs of the subject and a decrease in the acoustic resonance frequency may be indicative of an increase in the volume of gas in the lungs of the subject.

In some embodiments, the acoustic sensor may be a microphone or a speaker. In some embodiments, the acoustic sensor may be a speaker and the speaker may deliver the acoustic signal to the lungs of the subject.

In some embodiments, the acoustic response may be determined from a transfer function relating a current signal measured at the speaker in response to delivery of the acoustic signal to the lungs of the subject to a voltage signal applied to the speaker to cause the speaker to deliver the acoustic signal to the lungs of the subject.

In some embodiments, the apparatus may further comprise a mask configured to cover at least one of a mouth and a nose of the subject for acquisition of the measurement.

According to a second aspect, there is provided a method of operating an apparatus comprising a control unit to estimate a volume of liquid in the lungs of a subject. The method comprises acquiring a measurement indicative of one or both of a volume of gas in the lungs of the subject and a change in a volume of gas in the lungs of the subject. The method also comprises estimating the volume of liquid in the lungs of the subject from the acquired measurement.

According to a third aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, a volume of liquid in the lungs of a subject is estimated based on a measurement indicative of one or both of a volume of gas in the lungs of the subject and a change in a volume of gas in the lungs of the subject. In this way, it is possible to more accurately estimate a volume of liquid in the lungs of the subject using the relationship between a level of gas in an acoustic or fluidic flow system and a level of liquid in the acoustic or fluidic flow system, thereby providing an apparatus with improved reliability, and it is also possible to perform this estimation of the volume of liquid in the lungs in a simple, yet effective, manner. Since the relationship between a level of gas in an acoustic or fluidic flow system and a level of liquid in the acoustic or fluidic flow system can be used to estimate the volume of liquid in the lungs of a subject, the apparatus can be less obtrusive. The apparatus can be useful, for example, for users to monitor a status of chronic heart failure (CHF), chronic obstructive pulmonary disease (COPD), or any other condition or disease that is related to a volume of liquid in the lungs of a subject.

These and other aspects will be apparent from and elucidated with reference to the embodiment(s) described hereinafter.

BRIEF DESCRIPTION OF THE DRAWINGS

For a better understanding of the embodiments, and to show more clearly how they may be carried into effect, reference will now be made, by way of example only, to the accompanying drawings, in which:

Fig. 1 is a block diagram of an apparatus for estimating a volume of liquid in the lungs of a subject according to an embodiment;
Fig. 2(a) illustrates a graph of a relationship between a volume of gas added to a vessel and a measurement of pressure in the vessel;
Fig. 2(b) illustrates a graph of a relationship between a volume of liquid in the vessel and the measurement of the pressure in the vessel;
Fig. 3 illustrates a method of operating an apparatus comprising a control unit to estimate a volume of liquid in the lungs of a subject;
Fig. 4 illustrates a method of operating an apparatus comprising a control unit to estimate a volume of liquid in the lungs of a subject according to an example embodiment; and
Fig. 5 illustrates a method of operating an apparatus comprising a control unit to estimate a volume of liquid in the lungs of a subject according to another example embodiment.

DETAILED DESCRIPTION OF THE EMBODIMENTS

As noted above, there is provided an improved apparatus and method for estimating a volume of liquid in the lungs of a subject, which overcomes the existing problems.

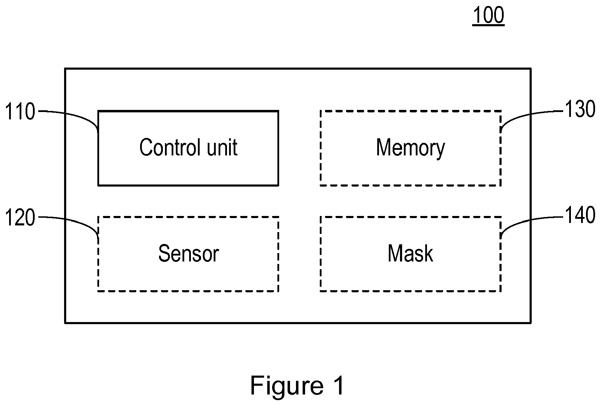

Fig. 1 shows an apparatus 100 for estimating a volume of liquid in the lungs of a subject according to an embodiment. The subject may, for example, be a patient, such as a patient with a chronic heart failure (CHF) condition and/or symptoms, or a patient with any other condition and/or symptoms, or any combination of conditions and/or symptoms, for which an estimation of the volume of liquid in the lungs can be useful. The apparatus 100 comprises a control unit 110.

Briefly, the control unit 110 is configured to acquire a measurement indicative of one or both of a volume of gas in the lungs of the subject and a change in a volume of gas in the lungs of the subject, and to estimate the volume of liquid in the lungs of the subject from the acquired measurement. As will be explained in further detail below, the acquired measurement may be one or more of a measurement of a volume of gas in the lungs of the subject, a measurement of a pressure in the lungs of the subject, an acoustic signal detected from the lungs of the subject, or any other measurement, or any combination of measurements, indicative of one or both of a volume of gas in the lungs of the subject and a change in a volume of gas in the lungs of the subject.

The control unit 110 can be implemented in numerous ways, with software and/or hardware, to perform the various functions described below. The control unit 110 may comprise one or more microprocessors or digital signal processor (DSPs) that may be programmed using software or computer program code to perform the required functions and/or to control components of the control unit 110 to effect the required functions. The control unit 110 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, as illustrated in Fig. 1, the apparatus 100 may also comprise one or more memories 130. Alternatively or in addition, one or more memories 130 may be external to (i.e. separate to or remote from) the apparatus 100. The memory 130 may be configured to store program code that can be executed by the control unit 110 to perform the method described herein. The one or more memories may comprise any type of memory. For example, the one or more memories may comprise one or more cache or system memories, which may include one or more volatile and/or non-volatile computer memories, such as any one or more random access memories (RAMs), static random access memories (SRAMs), dynamic random access memories (DRAMs), read-only memories (ROMs), programmable read-only memories (PROMs), erasable programmable read-only memories (EPROMs), electrically erasable programmable read-only memories (EEPROMs), or any other memory, or any combination of memories.

As mentioned earlier, the control unit 110 of the apparatus 100 is configured to estimate the volume of liquid in the lungs of the subject from the acquired measurement that is indicative of one or both of a volume of gas in the lungs of the subject and a change in a volume of gas in the lungs of the subject. In some embodiments where the measurement is indicative of a volume of gas in the lungs of the subject, the control unit 110 may be configured to perform the estimation of the volume of liquid in the lungs of the subject from the acquired measurement by comparing the acquired measurement to one or more reference values. For example, the one or more reference values may relate a measurement indicative of a volume of gas in the lungs of the subject with a volume of liquid in the lungs of the subject. In any of the embodiments described herein, the one or more reference values may, for example, include a measurement indicative of a volume of gas in the lungs when the volume of liquid in the lungs of the subject is zero, or close to zero (or when there is no liquid or a negligible volume of liquid in the lungs). In some embodiments, the reference values for measurements indicative of a volume of gas in the lungs of the subject maybe stored in the form of a look-up table with associated values for a volume of liquid in the lungs of the subject. The one or more reference values may, for example, be based on experimental data or a calibration procedure. The calibration procedure can be specific to the subject and/or performed in a certain setting (for example, in a medical facility such as a hospital). Thus, in this way, an absolute volume of liquid in the lungs of the subject can be estimated or determined.

In some embodiments where a measurement indicative of a change in a volume of gas in the lungs of the subject is acquired, the control unit 110 may be configured to estimate the volume of liquid in the lungs to increase when the change in volume of gas in the lungs is a decrease in the volume of gas in the lungs of the subject, and to estimate the volume of liquid in the lungs to decrease when the change in volume of gas in the lungs is an increase in the volume of gas in the lungs. This can, for example, be based on the assumption that the volume of liquid in the lungs of a subject is inversely proportional to the volume of gas in the lungs of the subject. This is based on the fact that when a liquid is added to (or arises in) the lungs of the subject, which are of a set volume, the volume of gas in the lungs decreases. Thus, $V_{liquid} = V_{lung} - V_{gas}$, where $V_{liquid}$ is the volume of liquid in the lungs of the subject, $V_{lung}$ is the total volume of the lungs of the subject and $V_{gas}$ is the volume of gas in the lungs of the subject. Thus, in this way, a relative volume of liquid in the lungs of the subject can be estimated or determined.

In some embodiments, as illustrated in Fig. 1, the apparatus 100 may further comprise a sensor 120. Alternatively or in addition, a sensor 120 may be external to (for example, remote or separate from) the apparatus 100. The sensor 120 can be configured to detect or acquire measurements relating to the lungs of the subject. The measurements can be any measurements indicative of one or both of a volume of gas in the lungs of the subject and a change in a volume of gas in the lungs of the subject. Thus, in embodiments where a sensor 120 detects or acquires measurements relating to the lungs of the subject, the control unit 110 of the apparatus 100 can be configured to acquire the measurement indicative of one or both of a volume of gas in the lungs of the subject and/or a change in a volume of gas in the lungs of the subject from the sensor 120.

In some embodiments, the sensor 120 (which the apparatus 100 may comprise or which may be external to the apparatus 100) may comprise a gas flow sensor, such as a spirometer or any other type of gas flow sensor suitable for measuring a movement of gas. In these embodiments, the control unit 110 can be configured to acquire, from the gas flow sensor, the measurement indicative of one or both of a volume of gas in the lungs of the subject and a change in a volume of gas in the lungs of the subject.

In some embodiments, for example, the gas flow sensor can be configured to acquire the measurement of the volume of gas in the lungs of the subject and the control unit 110 may thus be configured to acquire, from the gas flow sensor, the measurement of the volume of gas in the lungs of the subject. Thus, in these embodiments, the measurement indicative of one or both of the volume of gas in the lungs of the subject and a change in a volume of gas in the lungs of the subject can be acquired directly by the gas flow sensor. The control unit 110 can then estimate the volume of liquid in the lungs of the subject from the measurement acquired by the gas flow sensor.

For example, in embodiments where an absolute volume of liquid in the lungs of the subject is estimated or determined, the control unit 110 can be configured to estimate or determine the volume of liquid in the lungs of the subject by comparing the measurement of the volume of gas in the lungs to one or more reference values, as described above. In another example, in embodiments where a relative volume of liquid in the lungs of the subject is estimated or determined, a change in the volume of gas in the lungs of the subject can be indicative of a change in the volume of liquid in the lungs of the subject. Specifically, as described earlier, the control unit 110 can be configured to estimate the volume of liquid in the lungs to increase when the change in volume of gas in the lungs is a decrease in the volume of gas in the lungs of the subject and to estimate the volume of liquid in the lungs to decrease when the change in volume of gas in the lungs is an increase in the volume of gas in the lungs.

Alternatively or additionally, the sensor 120 (which the apparatus 100 may comprise or which may be external to the apparatus 100) may comprise a pressure sensor. In these embodiments, the control unit 110 can be configured to acquire, from the pressure sensor, the measurement indicative of one or both of a volume of gas in the lungs of the subject and a change in a volume of gas in the lungs of the subject. In some embodiments, for example, the pressure sensor can be configured to acquire a measurement of a pressure in the lungs of the subject and the control unit 110 may thus be configured to acquire, from the pressure sensor, the measurement of the pressure in the lungs of the subject. In these embodiments, the measurement of the pressure is the measurement indicative of one or both of the volume of gas in the lungs of the subject and a change in a volume of gas in the lungs of the subject. The pressure in the lungs of the subject for which a measurement is acquired may be in response to delivery (or injection) of a volume of gas to the lungs of the subject according to some embodiments.

In some embodiments, the measurement of the pressure in the lungs of the subject may comprise a measurement of a rate at which a threshold pressure is detected in the lungs of the subject, for example, in response to delivery (or injection) of the volume of gas to the lungs of the subject. For example, the measurement of the pressure in the lungs of the subject may comprise a pressure response when a (for example, controlled) volume of gas is delivered (or injected) to the lungs of the subject. The threshold pressure can, for example, be any preselected pressure that is equal to or less than a theoretical maximum pressure of fully inflated lungs or based on a theoretical maximum volume of gas required to fully inflate the lungs. The pressure response can be used to estimate the volume of liquid in the lungs since the lower the lung volume (i.e. the greater the ratio of liquid to gas in the lungs), the faster the pressure response (or the greater the rate at which pressure changes).

In these embodiments, the measurement of the rate at which the threshold pressure is detected is the measurement indicative of one or both of the volume of gas in the lungs of the subject and a change in a volume of gas in the lungs of the subject. For example, in embodiments where an absolute volume of liquid in the lungs of the subject is estimated or determined, the control unit 110 may be configured to estimate the volume of liquid in the lungs of the subject by comparing the acquired measurement of the rate at which the threshold pressure is detected to one or more reference values.

In another example, in embodiments where a relative volume of liquid in the lungs of the subject is estimated or determined, a change in the rate at which the threshold pressure is detected can be indicative of a change in the volume of gas in the lungs of the subject. Specifically, an increase in the rate at which the threshold pressure is detected may be indicative of a decrease in the volume of gas in the lungs of the subject, and a decrease in the rate at which the threshold pressure is detected may be indicative of an increase in the volume of gas in the lungs of the subject. In these embodiments, as described earlier, the control unit 110 can be configured to estimate the volume of liquid in the lungs to increase when the change in volume of gas in the lungs is a decrease in the volume of gas in the lungs of the subject and to estimate the volume of liquid in the lungs to decrease when the change in volume of gas in the lungs is an increase in the volume of gas in the lungs.

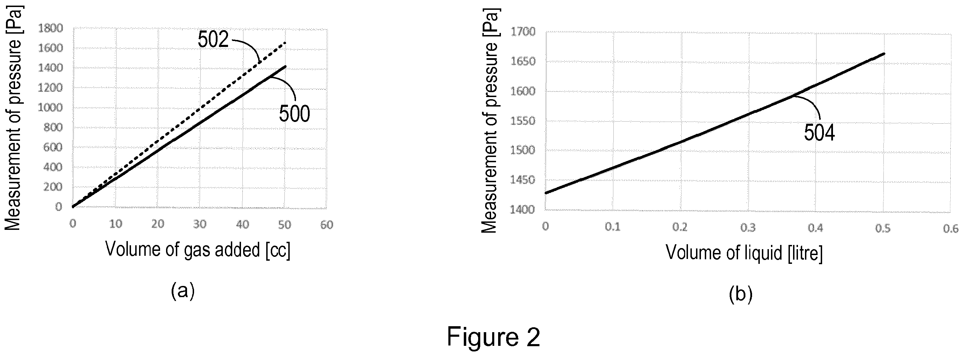

Fig. 2(a)-(b) illustrate graphs, according to an

**EP 3 456 260 A1**

example, of a relationship between a volume of gas added to a vessel and a measurement of the pressure in the vessel and a relationship between a volume of liquid in the vessel and the measurement of the pressure in the vessel respectively. The graphs illustrate the relationships described earlier, which apply to the lungs of the subject (where the lungs of the subject are the vessel). In this example illustration, gas is added to a 3.5 litre vessel. The gas is assumed to be an ideal gas at a constant temperature for the purpose of this example illustration. The 3.5 litres represents the capacity of the lung. The starting pressure is a normal atmospheric pressure of 1 bar.

The relationship between the volume of gas in the vessel and the pressure in the vessel where there is no liquid in the vessel is shown by line 500. The relationship between the volume of gas in the vessel and the pressure in the vessel where there is a (non-compressible) liquid in the vessel is shown by line 502. In this example illustration, 0.5 litres of liquid is present in the vessel. It can be seen from Fig. 2(a) that, when the volume of gas in the vessel increases, the pressure in the vessel also increases. It can further be seen from Fig. 2(a) that the pressure in the vessel increases faster (or at a greater rate) where there is a (non-compressible) liquid in the vessel (line 502) compared to where there is no liquid in the vessel (line 500), since the compressible gas volume decreases. Thus, the rate at which a threshold pressure is detected where there is a (non-compressible) liquid in the vessel (line 502) is higher than the rate at which the threshold pressure is detected where there is no liquid in the vessel (line 500).

Fig. 2(b) illustrates the pressure increase when 50cc of gas is added to the vessel (line 504). As can be seen from Fig. 2(b), the pressure increase is dependent on the volume of liquid in the vessel. In particular, as the volume of liquid in the vessel increases (i.e. as the ratio of liquid to gas in the lungs increases), the rate at which the pressure in the vessel increases is greater. Thus, as described earlier, the pressure response can be used to estimate the volume of liquid in the lungs (which corresponds to the vessel), since the faster the pressure response, the greater the volume of liquid in the lungs.

Alternatively or additionally, the sensor 120 (which the apparatus 100 may comprise or which may be external to the apparatus 100) may comprise an acoustic sensor. In these embodiments, the control unit 110 can be configured to acquire, from the acoustic sensor, the measurement indicative of one or both of a volume of gas in the lungs of the subject and a change in a volume of gas in the lungs of the subject. In some embodiments, for example, the acoustic sensor can be configured to acquire an acoustic signal detected from the lungs of the subject, the detected acoustic signal representing an acoustic response of the lungs of the subject, and the control unit 110 may thus be configured to acquire, from the acoustic sensor, the acoustic signal detected from the lungs of the subject. The acoustic response of the lungs of the subject (and thus the detected acoustic signal representing the acoustic response) may comprise one or more acoustic resonance frequencies. In these embodiments, the acoustic response (or one or more acoustic resonance frequencies of the acoustic response) of the lungs of the subject is the measurement indicative of one or both of the volume of gas in the lungs of the subject and a change in a volume of gas in the lungs of the subject. The acoustic signal may be detected from the lungs of the subject in response to delivery of an acoustic signal to the lungs of the subject according to some embodiments.

Examples of an acoustic sensor include, but are not limited to, a microphone, a speaker, or any other acoustic sensor, or any combinations of acoustic sensors. In embodiments where the acoustic sensor comprises a speaker, the speaker may be configured to deliver the acoustic signal to the lungs of the subject. In these embodiments, the acoustic response (or one or more acoustic resonance frequencies of the acoustic response) of the lungs of the subject may be determined from any one or more of a current signal measured at the speaker (for example, in response to delivery of the acoustic signal to the lungs of the subject) and a voltage signal applied to the speaker to cause the speaker to deliver the acoustic signal to the lungs of the subject.

For example, the control unit 110 maybe configured to determine the acoustic response (or one or more acoustic resonance frequencies of the acoustic response) from a transfer function relating the current signal measured at the speaker (for example, in response to delivery of the acoustic signal to the lungs of the subject) to the voltage signal applied to the speaker to cause the speaker to deliver the acoustic signal to the lungs of the subject. The person skilled in the art will be aware of suitable transfer functions that relate a current signal to a voltage signal, which can be employed. In some embodiments, the voltage signal applied to the speaker may be a frequency sweep or any other type of voltage signal suitable to cause the speaker to deliver the acoustic signal to the lungs of the subject. The acoustic response (or one or more acoustic resonance frequencies of the acoustic response) can be determined from the voltage-current transfer function since the acoustic response (or one or more acoustic resonance frequencies of the acoustic response) is typically associated with a minimum in a power function or a change in the sign in a phase change of the transfer function. In some embodiments, in addition to the speaker, one or more microphones can be placed to derive additional transfer functions related to sound pressure.

As mentioned earlier, the acoustic response (or one or more acoustic resonance frequencies of the acoustic response) is the measurement indicative of one or both of the volume of gas in the lungs of the subject and a change in a volume of gas in the lungs of the subject. For example, in embodiments where an absolute volume of liquid in the lungs of the subject is estimated or determined, the control unit 110 may be configured to estimate the volume of liquid in the lungs of the subject by comparing the acoustic response (or one or more acoustic resonance frequencies of the acoustic response) to one or more reference values.

In another example, in embodiments where a relative volume of liquid in the lungs of the subject is estimated or determined, a change in the acoustic response (or one or more acoustic resonance frequencies of the acoustic response) can be indicative of a change in the volume of gas in the lungs of the subject. Specifically, an increase in the acoustic response (or one or more acoustic resonance frequencies of the acoustic response) may be indicative of a decrease in the volume of gas in the lungs of the subject and a decrease in the acoustic response (or one or more acoustic resonance frequencies of the acoustic response) may be indicative of an increase in the volume of gas in the lungs of the subject. In these embodiments, as mentioned earlier, the control unit 110 can be configured to estimate the volume of liquid in the lungs to increase when the change in volume of gas in the lungs is a decrease in the volume of gas in the lungs of the subject and to estimate the volume of liquid in the lungs to decrease when the change in volume of gas in the lungs is an increase in the volume of gas in the lungs.

The relationship between an acoustic resonance frequency of an acoustic response of the lungs of the subject and the volume of gas in the lungs of the subject in some cases can be regarded as approximately similar to the relationship between the acoustic resonance frequency and the volume of gas within an acoustic system and this relationship could therefore be approximated using the Helmholtz resonance formula:

$$f = \frac{v}{2\pi}\sqrt{\frac{A}{Vl}}$$

wherein f is the acoustic resonance frequency determined from the transfer function using the current signal measured at the speaker and the voltage signal applied to the speaker in the manner described earlier, v is the speed of sound in a gas (e.g. air), A is the cross-sectional area of a neck of the acoustic system, V is the static volume of the cavity of the acoustic system, and l is the length of the neck of the acoustic system. For the purpose of estimating the volume of liquid in the lungs of the subject, the windpipe of the subject may be regarded as the neck of the acoustic system, and the lungs may be regarded as the cavity of the acoustic system.

The speed v of sound in a gas, for example in air, the cross-sectional area A of the windpipe of the subject (and/or a standard typical value of the cross-sectional area of windpipes), and the length l of the windpipe of the subject (and/or a standard typical value of the length of windpipes) may be predetermined or predefined and may be stored in the memory 130. Hence, using the Helmholtz resonance formula provided above, the static volume V of gas inside the acoustic system, i.e. the lungs of the subject in this embodiment, can be determined using the predetermined or predefined values stored in the memory 130 and the acoustic resonance frequency f can be determined from the transfer function.

Returning back to Fig. 1, as illustrated, the apparatus 100 may comprise a mask (or cap) 140 according to some embodiments. The mask (or cap) 140 can be configured to cover at least one of a mouth and a nose of the subject for acquisition of the measurement indicative of one or both of a volume of gas in the lungs of the subject and a change in a volume of gas in the lungs of the subject. The mask 140 may form a (closed) acoustic system together with the nose cavity and/or the mouth cavity, the windpipe, and the lungs of the subject so as to improve an accuracy of the estimation of the volume of liquid in the lungs of the subject. This can be useful, for example, in embodiments in which such estimation is performed using mathematical formulae (e.g. the Helmholtz resonance formula described earlier) that may be based on assumptions of rigidity of the cavity in the acoustic system and the stativity of the volume of gas in the acoustic system. In other embodiments, the apparatus 100 may instead comprise a device with a portion that can be inserted into the mouth of the subject to form a (closed) acoustic system. In these embodiments, the subject may also be provided with a nose piece that can be placed on the nose to block the flow of gas into and from the nose to form a closed acoustic system. Although examples have been provided for forming a (closed) acoustic system, the person skilled in the art will also be aware of other configurations.

In embodiments where the apparatus 100 comprises a sensor 120, the sensor 120 can be located at (for example, integrated in) the mask 140, located at (for example, integrated in) the device that can be inserted into the mouth, or configured to be placed (or positioned) on a part of the body of the subject. In some embodiments, the mask 140 or the device that can be inserted into the mouth maybe connected to the sensor 120 through a tube (not illustrated in Fig. 1). In these embodiments, the apparatus 100 may further comprise a pump (also not illustrated in Fig. 1) configured to deliver a volume of gas to the lungs of the subject through the tube and towards the mask 140 or the device that can be inserted into the mouth, and the sensor 120 may be located along the tube so as to acquire the measurement indicative of one or both of a volume of gas in the lungs of the subject and a change in a volume of gas in the lungs of the subject.

Although not illustrated in Fig. 1, in some embodiments, the apparatus 100 may also comprise a communications interface (or circuitry) for enabling the apparatus 100 to communicate with any components (such as sensors, memories, or any other components), interfaces and/or devices that are internal or external to the apparatus 100. The communications interface may communicate with any components, interfaces and/or devices wirelessly or via a wired connection. For example, in some embodiments, the control unit 110 may communicate with any sensors 120 and/or memories 130 via such a communications interface.

It will be appreciated that Fig. 1 only shows the components required to illustrate this aspect, and in a practical implementation the apparatus 100 may comprise additional components to those shown. For example, the apparatus 100 may comprise a battery or other power supply for powering the apparatus 100 or means for connecting the apparatus 100 to a mains power supply.

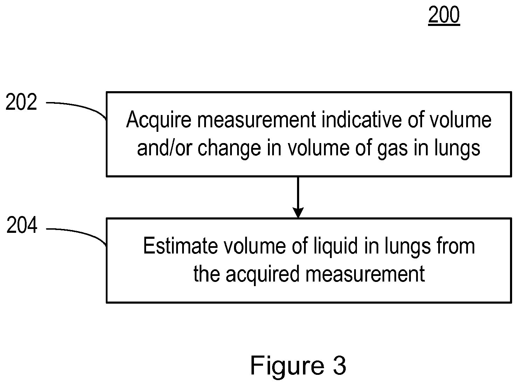

Fig. 3 illustrates a method 200 of operating the apparatus 100 described herein to estimate a volume of liquid in the lungs of a subject. The method 200 can generally be performed by or under the control of the control unit 110 of the apparatus 100.

Briefly, with reference to Fig. 3, the method comprises acquiring a measurement indicative of one or both of a volume of gas in the lungs of the subject and a change in a volume of gas in the lungs of the subject (at block 202 of Fig. 3). As described earlier with reference to Fig. 1, the control unit 110 of the apparatus 100 is configured to acquire the measurement and may, for example, acquire the measurement from one or more sensors 120 (which the apparatus 100 may comprise or which may be external to the apparatus 100). The method also comprises estimating the volume of liquid in the lungs of the subject from the acquired measurement (at block 204 of Fig. 3).

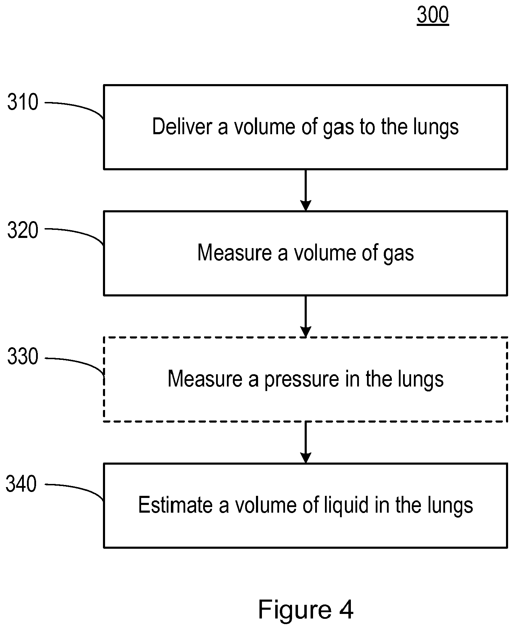

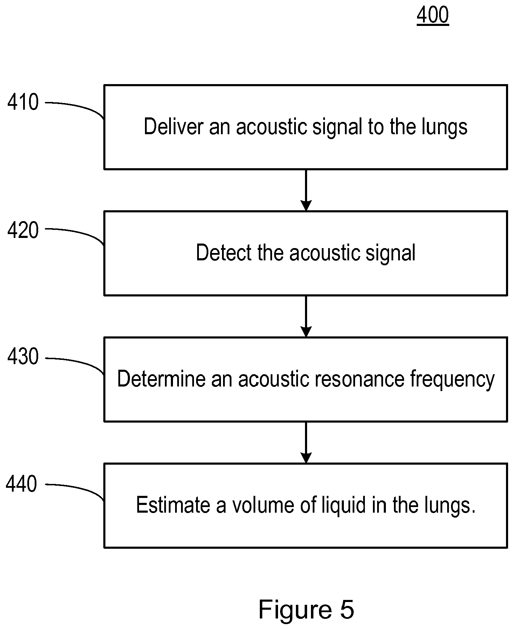

The control unit 110 of the apparatus 100 is configured to estimate the volume of liquid in the lungs of the subject from the acquired measurement using any of the techniques described earlier with reference to Fig. 1. Some of these techniques will now be described with reference to Figs. 4 and 5 as example embodiments of the method 200 of Fig. 3.

Fig. 4 illustrates a method 300 of operating the apparatus 100 described herein to estimate a volume of liquid in the lungs of a subject according to an example embodiment. The method 300 can generally be performed by or under the control of the control unit 110 of the apparatus 100.

With reference to Fig. 4, at block 310, a volume of gas is delivered to the lungs of the subject. The volume of gas may be delivered by a pump in the apparatus 100 or a pump provided external to the apparatus 100. In some embodiments, the volume of gas may be delivered through a tube towards a mask 140 of the apparatus 100 to the lungs of the subject, which can improve the efficiency of the apparatus 100 and the accuracy of measurements acquired in response to the delivery of the volume of gas.

At block 320 of Fig. 4, a volume of gas in the lungs of the subject is measured by the sensor 120 of the apparatus 100, which in this example embodiment comprises a gas flow sensor. The measurement of the volume of gas in the lungs of the subject can be indicative of one or both of the volume of gas in the lungs of the subject and a change in a volume of gas in the lungs of the subject. The gas flow sensor may be placed along a flow path of the volume of gas delivered to the lungs of the subject so as to acquire a reliable measurement indicative of one or both of a volume of gas in the lungs of the subject and a change in volume of gas in the lungs of the subject. The control unit 110 of the apparatus 100 can acquire the measurement of the volume of gas in the lungs of the subject from the gas flow sensor.

At block 330 of Fig. 4, which is an optional step in this example embodiment and which may be performed simultaneously with block 320, a measurement of pressure in the lungs of the subject is acquired by the sensor 120, which in this example embodiment also comprises a pressure sensor. The measurement of pressure is indicative of one or both of the volume of gas in the lungs of the subject and a change in a volume of gas in the lungs of the subject. The measurement of pressure in the lungs of the subject may, for example, comprise a measurement of a rate at which a threshold pressure is detected in the lungs of the subject in response to delivery of the volume of gas to the lungs of the subject at block 310. In this case, the measurement of the rate at which the threshold pressure is detected is indicative of one or both of the volume of gas in the lungs of the subject and a change in a volume of gas in the lungs of the subject. The control unit 110 of the apparatus 100 can acquire the measurement of the pressure (which may comprise the rate at which a threshold pressure is detected) in the lungs of the subject a from the gas flow sensor.

At block 340 of Fig. 4, the volume of liquid in the lungs of the subject is estimated by the control unit 110 of the apparatus 100 from at least one of the results from block 320 of Fig. 4 and block 330 of Fig. 4 (if performed). For example, if the measurement of pressure in the lungs at block 330 of Fig. 4 comprises a measurement of a rate at which a threshold pressure is detected in the lungs of the subject, an increase in the rate at which the threshold is detected may be indicative of a decrease in the volume of gas in the lungs of the subject and a decrease in the rate at which the threshold pressure is detected maybe indicative of an increase in the volume of gas in the lungs of the subject. Thus, the control unit 110 can estimate the volume of liquid in the lungs to increase when the change in volume of gas in the lungs is a decrease in the volume of gas in the lungs of the subject and decrease when the change in volume of gas in the lungs is an increase in the volume of gas in the lungs.

As another example, the volume of gas in the lungs of the subject measured at block 320 of Fig. 4 can be used by the control unit 110 to estimate or determine the volume of liquid in the lungs of the subject, for example, by comparing the measurement of the volume of gas in the lungs of the subject to one or more reference values, as described earlier.

Fig. 5 illustrates a method 400 of operating the apparatus 100 described herein to estimate a volume of liquid in the lungs of a subject according to another example embodiment. The method 400 can generally be performed by or under the control of the control unit 110 of the apparatus 100.

With reference to Fig. 5, at block 410, an acoustic signal is delivered to the lungs of the subject. More specifically, the acoustic signal may be provided by a sensor 120, which in this embodiment is a speaker. The speaker may be placed at a mask 140 of the apparatus 100, which is configured to cover at least one of a mouth and a nose of the subject, so as to provide an effective delivery of the acoustic signal to the lungs of the subject.

At block 420 of Fig. 5, the acoustic signal is detected by the speaker. Specifically, a current signal relating to the acoustic signal may be measured at the speaker in response to delivery of the acoustic signal to the lungs of the subject and a voltage signal applied to the speaker to cause the speaker to deliver the acoustic signal to the lungs of the subject may be measured or obtained.

At block 430 of Fig. 5, an acoustic resonance frequency of the detected acoustic signal at block 420 of Fig. 5 is determined, wherein the acoustic resonance frequency of the detected acoustic signal is indicative of one or both of the volume of gas in the lungs of the subject and change in a volume of gas in the lungs of the subject. Specifically, the acoustic resonance frequency may be determined from a transfer function relating the current signal from block 420 of Fig. 5 measured at the speaker to the measured voltage signal from block 420 of Fig. 5 or from the Helmholtz resonance formula as described earlier (for example, assuming that the acoustic system formed in part by the lungs of the subject is substantially similar to the acoustic system on which the Helmholtz resonance formula is based). The determined acoustic resonance frequency of the detected acoustic signal is indicative of one or both of the volume of gas in the lungs of the subject and a change in a volume of gas in the lungs of the subject.

At block 440 of Fig. 5, the volume of liquid in the lungs of the subject is estimated by the control unit 110 of the apparatus 100 from the result from block 430 of Fig. 5. Specifically, the volume of gas in the lungs of the subject can be used by the control unit 110 to estimate or determine the volume of liquid in the lungs of the subject.

For example, the control unit 110 may estimate or determine the volume of liquid in the lungs of the subject by comparing the acoustic resonance frequency determined at block 430 of Fig. 5 to one or more reference values. Alternatively or in addition, the control unit 110 may estimate or determine a change of the volume of gas in the lungs of the subject from a change in the acoustic resonance frequency. Specifically, an increase in the acoustic resonance frequency may be indicative of a decrease in the volume of gas in the lungs of the subject and a decrease in the acoustic resonance frequency may be indicative of an increase in the volume of gas in the lungs of the subject. Subsequently, from the change of volume of gas in the lungs of the subject, a change of the volume of liquid in the lungs of the subject can also be estimated, wherein the volume of liquid in the lungs of the subject is estimated to increase when the change in volume of gas in the lungs is a decrease in the volume of gas in the lungs of the subject, and the volume of liquid in the lungs of the subject is estimated to decrease when the change in volume of gas in the lungs is an increase in the volume of the gas in the lungs of the subject is estimated.

There is thus provided an improved apparatus and method for estimating a volume of liquid in the lungs of a subject, which overcomes the existing problems. The apparatus and method can be useful, for example, in monitoring a status of chronic heart failure (CHF), chronic obstructive pulmonary disease (COPD), or any other condition or disease that is related to a volume of liquid in the lungs of a subject.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for estimating a volume of liquid in the lungs of a subject, the apparatus (100) comprising a control unit (110) configured to:

   acquire a measurement indicative of one or both of a volume of gas in the lungs of the subject and a change in a volume of gas in the lungs of the subject; and
estimate the volume of liquid in the lungs of the subject from the acquired measurement.

2. The apparatus (100) as claimed in claim 1, wherein the volume of liquid in the lungs of the subject is estimated from the acquired measurement by comparing the acquired measurement to one or more reference values.

3. The apparatus (100) as claimed in claim 1 or 2, wherein:

the volume of liquid in the lungs of the subject is estimated to increase when the change in volume of gas in the lungs of the subject indicated by the acquired measurement is a decrease in the volume of gas in the lungs of the subject; and
the volume of liquid in the lungs of the subject is estimated to decrease when the change in volume of gas in the lungs of the subject indicated by the acquired measurement is an increase in the volume of gas in the lungs of the subject.

4. The apparatus (100) as claimed in any of the preceding claims, wherein the control unit (110) is configured to acquire the measurement from a gas flow sensor (120).

5. The apparatus (100) as claimed in any of claims 1, 2, or 3, wherein the control unit (110) is configured to acquire the measurement by acquiring, from a pressure sensor (120), a measurement of pressure in the lungs of the subject in response to a delivery of a volume of gas to the lungs of the subject, wherein the measurement of the pressure is indicative of one or both of the volume of gas in the lungs of the subject and a change in a volume of gas in the lungs of the subject.

6. The apparatus (100) as claimed in claim 5, wherein the measurement of pressure in the lungs of the subject comprises measurement of a rate at which a threshold pressure is detected in the lungs of the subject in response to delivery of the volume of gas to the lungs of the subject, and wherein the measurement of the rate at which the threshold pressure is detected is indicative of one or both of the volume of gas in the lungs of the subject and a change in a volume of gas in the lungs of the subject.

7. The apparatus (100) as claimed in claim 6, wherein:

an increase in the rate at which the threshold pressure is detected is indicative of a decrease in the volume of gas in the lungs of the subject;

and
a decrease in the rate at which the threshold pressure is detected is indicative of an increase in the volume of gas in the lungs of the subject.

8. The apparatus (100) as claimed in any one of claims 1, 2, or 3, wherein the control unit (110) is configured to acquire the measurement by acquiring, from an acoustic sensor (120), an acoustic signal detected in response to delivery of the acoustic signal to the lungs of the subject, wherein the detected acoustic signal represents an acoustic response of the lungs.

9. The apparatus (100) as claimed in claim 8, wherein the acoustic response comprises one or more acoustic resonance frequencies indicative of one or both of the volume of gas in the lungs of the subject and a change in a volume of gas in the lungs of the subject.

10. The apparatus (100) as claimed in claim 9, wherein:

an increase in the acoustic resonance frequency is indicative of a decrease in the volume of gas in the lungs of the subject; and
a decrease in the acoustic resonance frequency is indicative of an increase in the volume of gas in the lungs of the subject.

11. The apparatus (100) as claimed in any one of claims 8 to 10, wherein the acoustic sensor (120) is a speaker and wherein the speaker delivers the acoustic signal to the lungs of the subject.

12. The apparatus (100) as claimed in claim 11, wherein the acoustic response is determined from a transfer function relating a current signal measured at the speaker in response to delivery of the acoustic signal to the lungs of the subject to a voltage signal applied to the speaker to cause the speaker to deliver the acoustic signal to the lungs of the subject.

13. The apparatus (100) as claimed in any of the preceding claims, wherein the apparatus (100) further comprises a mask (140) configured to cover at least one of a mouth and a nose of the subject for acquisition of the measurement.

14. A method (200, 300, 400) of operating an apparatus (100) comprising a control unit (110) to estimate a volume of liquid in the lungs of a subject, the method comprising:

acquiring (202, 320, 330, 430) a measurement indicative of one or both of a volume of gas in the lungs of the subject and a change in a volume of gas in the lungs of the subject; and
estimating (204, 340, 440) the volume of liquid in the lungs of the subject from the acquired measurement.

15. A computer program product comprising a computer readable medium, the computer readable medium having computer readable code embodied therein, the computer readable code being configured such that, on execution by a suitable computer or processor, the computer or processor is caused to perform the method of claim 14.

Figure 1

Figure 2

<u>200</u>

202 — Acquire measurement indicative of volume and/or change in volume of gas in lungs

204 — Estimate volume of liquid in lungs from the acquired measurement

Figure 3

<u>300</u>

310 — Deliver a volume of gas to the lungs

320 — Measure a volume of gas

330 — Measure a pressure in the lungs

340 — Estimate a volume of liquid in the lungs

Figure 4

<u>400</u>

410 —⟨ | Deliver an acoustic signal to the lungs |

420 —⟨ | Detect the acoustic signal |

430 —⟨ | Determine an acoustic resonance frequency |

440 —⟨ | Estimate a volume of liquid in the lungs. |

Figure 5

Europäisches
Patentamt

European
Patent Office

Office européen
des brevets

## EUROPEAN SEARCH REPORT

Application Number

EP 17 19 1816

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 215 433 A2 (GATTINONI LUCIANO) 25 March 1987 (1987-03-25) * the whole document * ----- | 1-7, 13-15 | INV. A61B5/085 A61B5/091 A61M16/00 |
| X | WO 2013/181300 A1 (UNIV LELAND STANFORD JUNIOR [US]) 5 December 2013 (2013-12-05) * abstract * * paragraph [0080] * ----- | 1,8-12, 14,15 | |

TECHNICAL FIELDS
SEARCHED    (IPC)

A61B
A61M

The present search report has been drawn up for all claims

| Place of search | Date of completion of the search | Examiner |
|---|---|---|
| The Hague | 6 March 2018 | Van Dop, Erik |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 17 19 1816

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2018

| Patent document cited in search report | | Publication date | Patent family member(s) | | Publication date |
|---|---|---|---|---|---|
| EP 0215433 | A2 | 25-03-1987 | EP 0215433 A2 | | 25-03-1987 |
| | | | ES 2002326 A6 | | 01-08-1988 |
| | | | IT 1185906 B | | 18-11-1987 |
| | | | JP S6290136 A | | 24-04-1987 |
| | | | NO 863650 A | | 16-03-1987 |
| | | | US 4844085 A | | 04-07-1989 |
| WO 2013181300 | A1 | 05-12-2013 | US 2015150503 A1 | | 04-06-2015 |
| | | | WO 2013181300 A1 | | 05-12-2013 |

For more details about this annex : see Official Journal of the European Patent Office, No. 12/82